# EUROPEAN PATENT SPECIFICATION

(11) **EP 3 329 908 B1**
(45) Date of publication and mention of the grant of the patent: **29.01.2025**
(21) Application number: 17204906.6
(22) Date of filing: 01.12.2017
(51) Int. Cl.: A61K 9/20, A61K 31/496, A61K 9/00

(54) **ORALLY DISPERSIBLE TABLET FORM OF ARIPIPRAZOLE MONOHYDRATE FORMULATIONS**
ORAL DISPERGIERBARE TABLETTENFORM AUS ARIPIPRAZOL-MONOHYDRAT-FORMULIERUNGEN
COMPRIMÉ ORODISPERSIBLE DE FORMULATIONS SOUS FORME DE MONOHYDRATE D'ARIPIPRAZOLE

(30) Priority: 02.12.2016 TR 201617675
(43) Date of publication of application: 06.06.2018
(73) Proprietor: Sanovel Ilac Sanayi ve Ticaret A.S., Sisli - Istanbul (TR)
(72) Inventor: TÜRKYILMAZ, Ali, 34460 Istanbul (TR); PEHLIVAN AKALIN, Nur, 34460 Istanbul (TR); ÖNDER, Ramazan, 34460 Istanbul (TR); AYHAN, Merve, 34460 Istanbul (TR)
(74) Representative: Genç Ilhan, Oznur

(56) References cited:
- WO-A1-2014/173515
- WO-A1-2015/067313
- K VINOD KUMAR ET AL: "Formulation and evaluation of Orodispesible tablets of Aripiprazole tablets", INDIAN JOURNAL OF RESEARCH IN PHARMACY AND BIOTECHNOLOGY VINOD AND PRAGATPRINT), 31 December 2014 (2014-12-31), pages 2321 - 5674, XP055460347, Retrieved from the Internet <URL:http://www.ijrpb.com/issues/Volume%202_Issue%206/ijrpb%202(6)%208%20vinod%201473-1475.pdf> [retrieved on 20180316]

## Description

### Field of Invention

The present invention is related to an orally dispersible tablet formulation comprising aripiprazole monohydrate and at least one superdisintegrant.

### Background of Invention

Aripiprazole, with the chemical name 7-[4-[4-(2,3-dichlorophenyl)-1-piperazinyl]butoxy]-3,4-dihydrocarbostyril, has partial agonist effect on dopamine D2 receptors. Its chemical structure is illustrated with formula I given below.

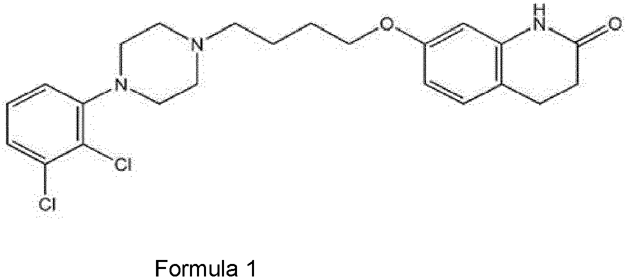

Aripiprazole monohydrate is marketed by Otsuka Pharmaceutical under the trademark ABILIFY DISCMELT^{®} which orally disintegrating tablets are available in 10 mg and 15 mg strengths. Inactive ingredients include acesulfame potassium, aspartame, calcium silicate, croscarmellose sodium, crospovidone, crème de vanilla (natural and artificial flavors), magnesium stearate, microcrystalline cellulose, silicon dioxide, tartaric acid, and xylitol. Colorants include ferric oxide (yellow or red) and FD&C Blue No. 2 Aluminum Lake.

Aripiprazole works in a slightly different way to other antipsychotic medicines. It acts on various receptors in the brain, particularly dopamine receptors and serotonin receptors. Dopamine and serotonin are natural compounds called neurotransmitters, and are involved in transmitting messages between brain cells. Psychotic illness is considered to be caused by disturbances in the activity of neurotransmitters (mainly dopamine) in the brain. Aripiprazole is thought to work mainly by stabilizing the dopamine activity in the brain. It is considered to be a dopamine D2 receptor partial agonist. A dopamine D2 receptor partial agonist has affinity toward the dopamine D2 receptor and an intrinsic activity that is less than the activity of the endogenous full dopamine agonist, that is, it can bind to the dopamine D2 receptor and cause a similar set of reaction but the magnitude of the reaction is smaller. As a consequence aripiprazole is more safe and tolerable in comparison to the existent typical and atypical antipsychotics.

The aripiprazole molecule is first disclosed in the patent EP367141 B1. In the same publication, aripiprazole was identified as being useful in the treatment of schizophrenia.

The patent application EP2001450 A2 discloses a formulation, describes a directly compressible composite prepared by co-processing a water-soluble excipient and calcium silicate. The present invention further describes the incorporation of the co-processed composite into a tablet formulation. The orally disintegrating tablets are of optimal mechanical strength and disintegrate within 60 seconds in the oral cavity.

The patent EP1145711 B1 discloses a formulation, comprising granules for the production of flash-melt pharmaceutical oral dosage forms comprising aripiprazole. In addition to one or more medicaments, the granules are composed of an excipient combination consisting of a superdisintegrant, a dispersing agent, a distributing agent, and a binder and may also include other conventional ingredients such as sweetening and flavoring agents.

The disclosure of WO 2015/067313 A1 pertains to orodispersible tablet compositions of aripiprazole. WO 2015/067313 A1 does not indicate any specific concerns with respect to the dissolution and disintegration properties of aripiprazole. The primary focus of WO 2015/067313 A1 is to achieve orodispersible tablets of aripiprazole through a straightforward manufacturing process. To address this need, WO 2015/067313 A1 suggests a directly compressible orodispersible tablet formulation of aripiprazole that includes lactose, at least one superdisintegrant, and at least one additional compressible diluent, specifically excluding alcoholic sugars and metal silicates.

Vinod Kumar et al. (Formulation and evaluation of orodispersible tablets of aripiprazole tablets. Indian Journal of Research in Pharmacy and Biotechnology, November-December 2014: 1473-1475) discusses mouth-dissolving tablets of aripiprazole. Notably, Vinod Kumar et al. does not reference the monohydrate form of aripiprazole. Vinod Kumar et al. examines the release profile of aripiprazole by adjusting the proportions of various excipients. According to Table 1 of Vinod Kumar et al., compositions of aripiprazole may include mannitol, microcrystalline cellulose, povidone, sodium starch glycolate, crospovidone, croscarmellose sodium, aspartame, lemon flavor, and magnesium stearate.

WO 2014/173515 A1, on the other hand, concerns orally disintegrating tablets of aripiprazole and seeks to enhance both the dissolution profile and stability of the tablet. WO 2014/173515 A1 recommends incorporating at least one water-soluble diluent and at least one water-insoluble diluent, with maltose and microcrystalline cellulose specifically mentioned to achieve these objectives.

In prior art, it is stated that use of calcium silicate with conventional equipments leads to discoloration of the final dosage form due to interaction of calcium silicate with some metals. Calcium silicate due to its hydrophobic and static nature results in blends with very poor flow properties causing weight and content variation during compression into tablets. Further, it also imparts a chalky taste to the dosage form.

In the present invention; an orally dispersible tablet formulation is provided that comprises aripiprazole monohydrate at 3% to 30% by weight, mannitol at 10% to 25% by weight, pregelatinized starch at 8% to 17% by weight, microcrystalline cellulose and guar gum at 5 %to 15% by weight, microcrystalline cellulose at 20% to 50% by weight, croscarmellose sodium at 4% to 20% by weight, sucralose at 0.1% to 4% by weight, Citric acid at 0.1% to 0.7% by weight, vanilla-flavored at 0.5% to 3% by weight, colloidal silicon dioxide 0,05% to 3% by weight, sodyum stearil fumarate 1% to 5% by weight, red iron oxide 0,0001% to 3% by weight.

At the present invention, orally dispersible tablet formulations of aripiprazole monohydrate do not comprise calcium silicate in contrast to prior art and by using croscarmellose sodium and mannitol, stability problems are solved and surprisingly better stability is gained.

In comparison to prior art, at present invention croscarmellose sodium is used as superdisintegrant instead of crospovidone. ODT formulated using croscarmellose sodium which has a concentration up to 10% showed better friability, disintegrating time, wetting time and dissolution profile than ODT formulated with low-substituted hydroxypropylcellulose, sodium starch glycolate, especially crospovidone.

At the present invention, an orally dispersible tablet can be rapidly disintegrated in the mouth. Further the formulations of the present invention overcome the problems of low disintegration, bitter taste and stability.

### Detailed Description of Invention

The main object of the present invention is to provide a formulation having a high mechanical resistance and low friability so the orally dispersible tablet can be rapidly disintegrated in the mouth.

Another object of the present invention is to provide the orally dispersible tablet of aripiprazole with enhanced taste. Aripiprazole has a bitter taste and there is a need to mask its taste in an oral solution to increase patient compliance. Flavoring combination with sweetening agents are used for this purpose.

Another object of the present invention is to obtain selection of excipients in a certain ratio has more importance to provide high bioavailability and avoidance of first pass metabolism.

The orally dispersible tablet provides for patients who have difficulty in swallowing, such as old people, infants, patients with mental problems and non-cooperative patients, as well as the population in general; since it makes it possible for the drug to be administered without the need for water. The orally dispersible tablet can be rapidly disintegrated in the mouth.

The orally dispersible tablet formulations of the present invention are characterized by excellent pharmacotechnical properties, such as flowability, compressibility and homogeneity. In more detail, the solid dosage forms of the present invention exhibit excellent pharmacotechnical characteristics including disintegration times, dissolution rates, hardness, friability, as well as stability. The orally dispersible tablet formulation of the present invention having also a high mechanical resistance and low friability presents a friability no greater than 1%, preferably no greater than 0.8%.

The hardness of the tablet has effect on the disintegration time. Therefore it is desirable to ensure the orally dispersible tablet which is soft enough to reach the desirable disintegration time at the desired time period and hard enough to overcome the negative effects of blistering process. These conditions are surprisingly provided in a range from 5 to 70 N, particularly it is 10 to 35N.

The selection of excipients has more importance to obtain the ideal disintegrating time during the shelf life. Especially, the choice of the disintegrant has a major role in the manufacture of the orally disintegrating tablets since it has an impact on both hardness and mouth feel of the composition. Therefore, the choice of a suitable disintegrant and an optimal use level are critical to ensure a high disintegration rate. Some disintegrants, also known as superdisintegrants, are particularly effective in inducing rapid tablet disintegration due to combined effect of swelling and water absorption by the formulation. Due to swelling of superdisintegrants, the wetted surface of the carrier increases thus promotes the wettability and dispersibility of the system and enhances the disintegration and dissolution.

The orally dispersible tablet formulation comprises aripiprazole monohydrate, croscarmellose sodium in an amount of between 4% to 20% by weight and at least one pharmaceutically acceptable excipient.

According to the present invention, the total amount of aripiprazole monohydrate in the composition is 3% to 30% by weight, preferably 5% to 15% by weight.

Furthermore, it has unexpectedly found that in this orally disintegrating composition having a weight ratio of aripiprazole monohydrate to croscarmellose sodium in the range of between 0.01-10.0, preferably the range of between 0.05-5.0, has a synergistic effect over the disintegration time.

According to the present invention, the superdisintegrant is croscarmellose sodium.

According to the present invention, the amount of croscarmellose sodium is present about 4 to 20% by weight of total composition.

Present invention, also offers better stability, taste and disintegration rate of orally disintegrating tablet formulations of aripiprazole monohydrate. According to present invention, orally dispersible tablet formulations of aripiprazole monohydrate does not comprise calcium silicate in contrast to prior art and by using croscarmellose sodium stability problems are solved and surprisingly better stability is gained.

Furthermore, croscarmellose sodium has physical and chemical properties that make it ideal for constituting the appropriate disintegrant for this invention. This helps croscarmellose sodium to dissolve easily and quickly in a little amount of water or saliva and makes its disintegrating rate much faster than other related excipients. The formulation disintegrates in the oral cavity in maximum 40 seconds, preferably in 30 seconds, more preferably in 15 to 20 seconds.

The orally dispersible tablet formulation comprises at least one pharmaceutically acceptable excipient which are selected from disintegrants, fillers, binders, glidants, lubricants, coloring agents, flavouring agents, sweetening agents and pH regulators or mixtures thereof.

According to the present invention; the disintegrant is mannitol.

According to the present invention; the binders are pregelatinized starch and microcrystalline cellulose and guar gum (Avicel CE15), the filler is microcrystalline cellulose.

The specific combination of croscarmellose sodium and microcrystalline cellulose has a synergistic effect over the disintegration time and mechanical strength of the orally disintegrating tablet formulation.

According to one embodiment of the invention, croscarmellose sodium is very effective excipient in the preparation of the orally disintegrating tablets. It has been surprisingly found that the object of the present invention is achieved when microcrystalline cellulose and croscarmellose sodium are used in a certain ratio. Consequently, the ratio of croscarmellose sodium to microcrystalline cellulose may be in the range of 0.01-10.0 by weight. Said ratio provides adequate disintegrating properties for the tablets of the present invention but also stabilizes the active pharmaceutical ingredient which does not degrade to unwanted impurities.

According to this embodiment of the invention, the weight ratio of croscarmellose sodium to microcrystalline cellulose is 0.01-10.0, preferably the ratio is 0.05-5.0.

According to the present invention; the glidant is silica colloidal dioxide.

The lubricant is used in this invention as an additional excipient that can affect the performance of the orally dispersible tablet formulation. According to the present invention; the lubricant for the composition is sodium stearyl fumarate.

Aripiprazole has a bitter taste and there is a need to mask its taste in an oral solution to increase patient compliance. Flavoring combination with sweetening agents are used for this purpose.

According to the present invention; the flavouring agent is vanilla.

According to the present invention; the coloring agent is red iron oxide.

According to the present invention; the pH regulator is citric acid.

According to one embodiment of the invention is the orally dispersible tablet formulation comprising the following ingredients; aripiprazole monohydrate at 3% to 30% by weight, mannitol at 10% to 25% by weight, pregelatinized starch at 8% to 17% by weight, microcrystalline cellulose and guar gum at 5 %to 15% by weight, microcrystalline cellulose at 20% to 50% by weight, croscarmellose sodium at 4% to 20% by weight, sucralose at 0.1% to 4% by weight, Citric acid at 0.1% to 0.7% by weight, vanilla-flavored at 0.5% to 3% by weight, colloidal silicon dioxide 0,05% to 3% by weight, sodium stearil fumarate 1% to 5% by weight, red iron oxide 0,0001% to 3% by weight.

According to one embodiment of the invention, the orally dispersible tablet formulation comprising aripiprazole monohydrate is prepared by using direct compression method.

Direct compression method is the most commonly used method in producing ODT because it is the easiest method in tablet manufacturing, can use conventional manufacturing instrument, short procedure, relatively cheap, can be loaded with thermolabile and moisture sensitive drugs and can be made into high dose.

Another embodiment of the present invention provides a process for preparing the orally dispersible tablet formulation according to the present invention, this method comprising the steps of;
a. mixing aripiprazole monohydrate and microcrystalline cellulose pH 112 for 5 minutes
b. adding microcrystalline cellulose and guar gum (Avicel CE15), mannitol (DC400), pregelatinized starch, croscarmellose sodium, sucralose to step (a) mixture and further mixing for 10 minutes
c. adding citric acid, colloidal silicon dioxide, sodyum stearil fumarate, vanilla-flavored, red iron oxide to step(b) mixture and further mixing for 5 minutes.
d. compressing the powder mixture into tablets

### Example

| **Content** | Amount (% by weight of the total) |
|---|---|
| aripiprazole monohydrate | 5.0-15.0 |
| mannitol (DC400) | 15.0 - 22.0 |
| pregelatinized starch | 10.0 - 14.0 |
| microcrystalline cellulose and guar gum (Avicel CE15) | 8.0-13.0 |
| microcrystalline cellulose (PH 112) | 25.0 - 35.0 |
| croscarmellose sodium | 9.0-15.0 |
| sucralose | 0.5-2.0 |
| citric acid | 0.15- 0.50 |
| vanilla-flavored | 1.0 - 2.0 |
| colloidal silicon dioxide | 0.1 -1.0 |
| sodyum stearil fumarate | 2.0 - 3.0 |
| red iron oxide | 0.0001 - 1.00 |

Process for preparing the orally dispersible tablet formulation comprising;
a. mixing aripiprazole monohydrate and microcrystalline cellulose pH 112 for 5 minutes
b. adding microcrystalline cellulose and guar gum (Avicel CE15), mannitol (DC400), pregelatinized starch, croscarmellose sodium, sucralose to step (a) mixture and further mixing again for 10 minutes
c. adding citric acid, colloidal silicon dioxide, sodium stearil fumarate, vanilla-flavored, red iron oxide to step(b) mixture and further mixing for 5 minutes.
d. compressing the powder mixture into tablets.

## Claims

1. An orally dispersible tablet formulation comprising:
a. 3% to 30% aripiprazole monohydrate
b. 10% to 25% mannitol
c. 8% to 17% pregelatinized starch
d. 5 %to 15% microcrystalline cellulose and guar gum
e. 20% to 50% microcrystalline cellulose
f. 4% to 20% croscarmellose sodium
g. 0.1% to 4% sucralose
h. 0.1% to 0.7% citric acid
i. 0.5% to 4% vanilla-flavored
j. 0,05% to 3% colloidal silicon dioxide
k. 1% to 5% sodium stearil fumarate
l. 0,0001% to 3% red iron oxide
by weight of total tablet.

2. Process for preparing the orally dispersible tablet according to claim 1, comprising the following steps;
a. mixing aripiprazole monohydrate and microcrystalline cellulose for 5 minutes
b. adding microcrystalline cellulose and guar gum, mannitol, pregelatinized starch, croscarmellose sodium, sucralose to step (a) mixture and further mixing again for 10 minutes
c. adding citric acid, colloidal silicon dioxide, sodium stearil fumarate, vanilla-flavored, red iron oxide to step(b) mixture and further mixing for 5 minutes.
d. compressing the powder mixture into tablet.

## Patentansprüche

1. Oral dispergierbare Tablettenformulierung, umfassend:
a. 3% bis 30% Aripiprazol-Monohydrat
b. 10% bis 25% Mannitol
c. 8% bis 17% vorverkleisterte Stärke
d. 5 % bis 15 % mikrokristalline Cellulose und Guarkernmehl
e. 20% bis 50% mikrokristalline Cellulose
f. 4% bis 20% Croscarmellose-Natrium
g. 0,1% bis 4% Sucralose
h. 0,1% bis 0,7% Zitronensäure
i. 0,5% bis 4% mit Vanillegeschmack
j. 0,05% bis 3% kolloidales Siliziumdioxid
k. 1% bis 5% Natriumstearilfumarat
l. 0,0001% bis 3% rotes Eisenoxid
bezogen auf das Gewicht der gesamten Tablette.

2. Verfahren zur Herstellung der oral dispergierbaren Tablette nach Anspruch 1, umfassend die folgenden Schritte;
a. Mischen von Aripiprazol-Monohydrat und mikrokristalliner Cellulose für 5 Minuten
b. Zugabe von mikrokristalliner Cellulose und Guarkernmehl, Mannitol, vorverkleisterter Stärke, Croscarmellose-Natrium und Sucralose zur Mischung aus Schritt (a) und erneutes Mischen für 10 Minuten
c. Zugabe von Zitronensäure, kolloidalem Siliciumdioxid, Natriumstearilfumarat, Vanillegeschmack und rotem Eisenoxid zur Mischung aus Schritt b) und weiteres Mischen für 5 Minuten.
d. Komprimieren der Pulvermischung zu einer Tablette.

## Revendications

1. - Formulation de comprimé orodispersible comprenant :
a. de 3 % à 30 % d'aripiprazole monohydraté ;
b. de 10 % à 25 % de mannitol ;
c. de 8 % à 17 % d'amidon prégélatinisé ;
d. de 5 % à 15 % de cellulose microcristalline et de gomme de guar ;
e. de 20 % à 50 % de cellulose microcristalline ;
f. de 4 % à 20 % de croscarmellose sodique ;
g. de 0,1 % à 4 % de sucralose ;
h. de 0,1 % à 0,7 % d'acide citrique ;
i. de 0,5% à 4 % d'arôme de vanille ;
j. de 0,05 % à 3 % de dioxyde de silicium colloïdal ;
k. de 1 % à 5 % de fumarate de stéaryle et de sodium ;
l. de 0,0001 % à 3 % d'oxyde de fer rouge
en poids du comprimé total.

2. - Procédé de préparation du comprimé orodispersible selon la revendication 1, comprenant les étapes suivantes :
a. mélanger l'aripiprazole monohydraté et la cellulose microcristalline pendant 5 minutes ;
b. ajouter la cellulose microcristalline et la gomme de guar, le mannitol, l'amidon prégélatinisé, la croscarmellose sodique, le sucralose au mélange de l'étape (a) et continuer à mélanger encore pendant 10 minutes ;
c. ajouter l'acide citrique, le dioxyde de silicium colloïdal, le fumarate de stéaryle et de sodium, l'arôme de vanille, l'oxyde de fer rouge au mélange de l'étape (b) et continuer à mélanger pendant 5 minutes ;
d. comprimer le mélange pulvérulent en comprimé.
